# EUROPEAN PATENT APPLICATION

(11) **EP 3 399 299 A1**
(43) Date of publication of application: **07.11.2018**
(21) Application number: 17169537.2
(22) Date of filing: 04.05.2017
(51) Int. Cl.: G01N 21/21, G01N 33/00, G01N 33/28, G01N 21/552

(54) **GAS SENSOR EMPLOYING POLARIZATION, GAS DETECTION SYSTEM AND METHOD EMPLOYING THE SAME**

(71) Applicant: ABB Schweiz AG, 5400 Baden (CH)
(72) Inventor: MÜLLER, Georg, 8152 Glattpark (CH); GREMAUD, Robin, 8048 Zürich (CH); VAN MECHELEN, Jacobus Lodevicus Martinus, 8104 Regensdorf (CH)
(74) Representative: ABB Patent Attorneys

(57) **Abstract**

A hydrogen sensing system (1) for detecting hydrogen in a medium (2) to be monitored, the system comprising a light emitting unit (24) emitting light with two polarization components having a first intensity ratio between the polarization components, an optical element (12, 22), a sensor layer (11) which is attached, with a first face, to a sensing face (12b, 22b) of the optical element, wherein the sensor layer (11) is configured to be in contact, at a second face opposite to the optical element, with the medium (2), and to have a refractive index which is dependent on a hydrogen partial pressure in the medium at the sensor layer (11), a light detection unit (19) configured to detect the intensity of each of the two polarization components of the detected light, and wherein a control unit (20) is configured to determine a gas partial pressure at the sensor layer (11) on the basis of a ratio of the intensities of the polarization components of the reflected light as detected by the light detection unit (19).

## Description

### Field

Aspects of the present disclosure relate to a gas sensor, particularly to a hydrogen sensor, a gas sensing system employing the same, and to an electrical device including such a detection system. In particular, aspects relate to a hydrogen sensor and a hydrogen sensing or detection system with such a sensor, having improved properties and being suitable for liquid-filled electrical equipment.

### Technical background

Insulation-liquid-filled electrical equipment, such as oil-filled shunt reactors, bushings, and especially transformers such as power and distribution transformers, are filled with insulation liquid, in particular oil, for cooling and electrical insulation purposes. Faults inside the electrical equipment as well as degradation of the insulation liquid and of other insulation components such as insulation paper provided within the electrical equipment can form decomposition gases which mainly dissolve into the liquid. This is valid for equipment employing both mineral oil and oil from natural sources.

It is important to detect such faults, errors and degradations early, since especially transformers are important components of the electrical grid, and their failure can be very costly. Hence, a transformer is supposed to operate continuously and as error-free as possible over many years or even decades.

The quantity and composition of the decomposition gases is dependent on the underlying defect: A large fault with high energy content, such as rapid overheating or arcing, causes large amounts of gas produced in a short period of time, whereas the amount of gas produced by a small fault may be relatively smaller. Also, the relative concentrations of the different gasses dissolved might indicate the specific type of fault. Thus, if the nature and amount of individual gases dissolved in the insulation liquid are known, the occurrence of a change of the concentration of a specific gas in the oil can be used to identify an electrical fault in the equipment. It is known that one of the most important indicators for electrical failure in oil insulated transformers is the occurrence of hydrogen gas dissolved in the oil, which is for example produced at a faulty portion of an insulation of a winding of the transformer, by thermal or electrical decomposition of the oil. For this reason, it is desirable that such errors, which may eventually cause complete failure of the transformer, can be detected as early as possible by identifying a rise in hydrogen concentration. This should ideally be possible at a stage when appropriate counter-measures may still be taken before serious and potentially costly malfunction occurs.

At a very early stage of such an electrical fault, only a very small amount of hydrogen gas may be produced, which dissolves in the oil and thus a concentration of dissolved hydrogen builds up in the oil over a longer period of time - whereby the hydrogen concentration in the oil may, at least during an early phase of the failure, even be below a threshold at which it can be detected with most known detection methods.

For detecting even small traces of hydrogen in transformer oil, on-line hydrogen monitoring devices are known which include thin-film based fiber-optic sensors, wherein a sensing material, which is typically coated on a fiber end, changes its reflectivity upon an exposure to hydrogen dissolved in the oil surrounding the sensor. One such system for detecting hydrogen gas is described as an optical switching device in WO 2007 049965 A1.

Further types of hydrogen sensors use light reflection off a palladium layer or a similar layer under one or more angles which deviate from normal incidence. For example, in one configuration, palladium (Pd) is coated on an optical prism, see PL203784 B1, or Chadwick, B. and M. Gal: "Enhanced optical detection of hydrogen using the excitation of surface plasmons in palladium", Applied Surface Science 68(1), 1993, p. 135-138. Palladium may also be coated around an optical multimode fiber, such as described by Bévenot, X., A. Trouillet, et al: "Surface plasmon resonance hydrogen sensor using an optical fibre." Measurement Science and Technology 13(1), 2002, p. 118.

US 7116421 B2 discloses a hydrogen sensor for hydrogen gas detection. It involves providing a gas sample, generating modulated optical excitation sources, bringing the optical excitation source to the side of a metalized thin pyroelectric film, and detecting via a lock-in amplifier circuit the coherent differential signal resulting from thermoreflectance and thermoabsorptance changes in the film when exposed to hydrogen gas.

DE 101 45 719 A1 discloses an optical hydrogen sensor which comprises an optically transparent substrate with a first surface, a hydrogen-sensitive layer containing or consisting of a hydrogen-sensitive metal, metal oxide and/or polymer formed on the first surface, a light source and an optical detector. A separating layer made from a dielectric is arranged between the substrate and the hydrogen-sensitive layer. Light from the light source undergoes total reflection at the boundary between the transparent substrate and the dielectric.

The known solutions leave room for improvement. Hence, there is a need for the present invention.

### Summary of the invention

In view of the above, a hydrogen sensing system according to claim 1, a method of determining a hydrogen concentration in a medium according to claim 10, and a use according to claim 15 are provided.

According to an aspect, a sensing system for detecting hydrogen in a medium is provided. The system includes a light emitting unit emitting light with two polarization components having a first intensity ratio between the polarization components; an optical element having a sensor layer which is attached, with a first face, to a sensing face of the optical element, wherein the sensor layer is configured to be in contact, at a second face opposite to the optical element, with a medium to be monitored, and to have a refractive index which is dependent on a hydrogen partial pressure at the sensor layer, a light detection unit configured to detect the intensity of each of the two polarization components of the detected light; a control unit operatively coupled to the light detection unit, wherein light emitted by the light emitting unit is guided into the optical element and is partially reflected at an angle of incidence larger than 0°, at the boundary between the sensor layer and the optical element, and wherein the control unit is configured to determine a gas partial pressure at the sensor layer on the basis of a ratio of the intensities of the polarization components of the reflected light as detected by the light detection unit.

According to a second aspect, a method for detecting hydrogen in a medium is provided. The method includes: providing a sensing system according to the first aspect; coupling light having two polarization components, having a first intensity ratio between the polarization components, into an optical element; reflecting the light at a boundary between a sensing face of the optical element and a sensor layer which is in contact with a medium to be monitored and has a refractive index being dependent on a hydrogen partial pressure in the medium; guiding the reflected light out of the optical element and onto a light detection unit configured to detect the intensity of each of the two polarization components of the detected light; measuring the intensities of the two polarization components; determining a gas partial pressure in the medium on the basis of a ratio of the intensities of the two polarization components of the detected light.

According to a further aspect, the use of a sensing system is provided. The system includes a light emitting unit, an optical element with a hydrogen-sensitive sensor layer attached to a sensing face of the optical element, a light detection unit and a control unit, to determine a hydrogen partial pressure in a medium in contact with the sensor layer, by employing a measurement of the intensities of two polarization components of light reflected, at an angle of incidence being larger than 0°, at a boundary between the optical element and the sensor layer, wherein a ratio of the intensities of the two polarization components of the reflected light is used to determine the hydrogen concentration in the medium.

Further advantages, features, aspects and details that can be combined with embodiments described herein are evident from the dependent claims, the description and the drawings.

In the proposed sensing system and method, parameters such as intensity fluctuations of the light source as well as changes in the characteristics of the optical couplings in the system et cetera are automatically taken into account.

In comparison to many known optical sensor configurations using a transparent substrate with an attached sensing layer, in embodiments reflected light powers for both s- and p-polarized light vary with hydrogen uptake. Embodiments do not cover working in an angular range which would result in a total reflection of the impinging light wave. The angle of incidence may be chosen to be in the regime which encompasses a range larger than 0°.

While in this disclosure, most embodiments and examples are directed to a hydrogen sensor and hydrogen sensing system and method, employed for detecting hydrogen in a fluid being transformer oil, these are only exemplary, non-limiting examples. In embodiments, the concept may also be employed for the detection of other gases than hydrogen, and in other fluids than transformer oil. As an example, concentrations of a gas other than hydrogen in a gas mixture - or a liquid - may be detected.

### Brief description of the Figures

More details will be described in the following with reference to the figures, wherein
Fig. 1 is a schematic view of a hydrogen sensing system according to embodiments;
Fig. 2 is a schematic view of the ratio of Pp (light power of reflected p-polarized light) to Ps (light power of reflected s-polarized light) for various angles of incidence, according to embodiments;
Fig. 3A,3B show schematic cross-sectional views on different shapes of non-limiting optical elements according to embodiments;
Fig. 4 shows the reflected light powers (normalized to incident light power) for s-polarized and p-polarized light components according to an exemplary embodiment at different hydrogenation levels of the sensor layer;
Fig. 5 depicts the sensitivity of a sensor, according to embodiments, to changes of the real part and the imaginary part of the refractive index of a sensor layer at different angles of incidence.

### Detailed Description of Aspects of the Invention

As used herein, the term "fluid" is intended to be both representative for gases and liquids. It is, however, mainly used to be representative of an insulation liquid, particularly an oil, which is part of the insulation and/or cooling system of an electrical device, such as a power transformer.

As used herein, the term "angle of incidence" is intended to mean, in terms of a light beam or wave impinging on a surface, the angle between the light beam or wave and a normal direction on the surface, meaning that a light beam impinging the surface at a normal direction has an angle of incidence of 0°.

As used herein, the terms "s-polarization" and "p-polarization" are intended to be defined with respect to the plane of incidence - which is the plane made by the incoming beam propagation direction and the vector perpendicular to a surface, in other words, the plane in which the ray travels before and after reflection at the surface. The component of the electric field parallel to this plane is termed the p-polarization component, and the component perpendicular to this plane is termed the s-polarization component. Polarized light with its electric field along the plane of incidence is thus denoted p-polarized, while light whose electric field is normal to the plane of incidence is called s-polarized.

As used herein, the term "wavelength of operation" is intended to be the wavelength at which the light source emits light, or, in case of a broad emission spectrum, as a wavelength representative for the emitted light spectrum, such as the center of gravity of the spectrum or a wavelength specified by the manufacturer of the light source.

As used herein, the expression "the ratio" in conjunction with the measured intensities of two polarization components of reflected light means either the mathematical ratio between the intensities of the two components, or can as well mean to calculate a difference of the two intensities, divided by the sum of the two intensities, which by definition also yields "the ratio". In embodiments described herein, both variants/meanings are equally applicable. It goes without saying that the skilled person may find other ways of calculating a "ratio", in the broadest possible sense, from and of the two measured intensities, without departing from the technical meaning and application of this process, as described herein. Hence, such variations are regarded to also fall into the scope of the present disclosure.

In the following, some aspects of the invention are described in detail. Aspects and parts of aspects are independent of each other and can be combined in any manner. For example, any aspect or embodiment described in this document can be combined with any other aspect or embodiment, as long as the combinations achieved are technically feasible.

First, some general possible aspects relating to the hydrogen sensor are described. The sensor is suitable and adapted for sensor a status condition of an insulation-liquid-filled electrical equipment. Herein, electrical equipment refers to any equipment such as shunt reactors, bushings and transformers. The invention is particularly suited for the insulation liquid being insulation oil, be it on a mineral basis or from organic sources, such as palm oil. The invention is further particularly suited for the electrical equipment being a transformer, such as a power or distribution transformer, and even more particularly for an oil-filled transformer.

The status condition of the electrical equipment is herein expressed by the hydrogen content (or hydrogen concentration) of the insulation liquid, which is a reliable indicator of various conditions, in particular fault conditions. The hydrogen content is defined as the amount of hydrogen dissolved in the insulation liquid (e.g., in ppm). A hydrogen sensitive layer (henceforth also called sensor layer) of an optical sensor is arranged in communication with the fluid (insulation liquid, oil), and is preferably immersed in the insulation liquid, so that the amount of hydrogen dissolved in the insulation liquid results in a characteristic partial pressure of hydrogen at the optical sensor, this partial pressure being a function of the hydrogen content (in ppm) in the insulation liquid; alternatively, the sensor layer can also be separated from the insulation liquid by a hydrogen permeable membrane. This correlation may depend on additional parameters such as the temperature of the insulation liquid and/or of the hydrogen sensitive layer, and on the type of oil used in the transformer. Herein, the term "hydrogen" may refer to hydrogen molecules or atoms (which may be radicals). As used herein, the sensor layer "being in communication with a fluid" means that the gaseous components of interest present in the fluid, in particular hydrogen, may reach the sensor layer, even if other layers for catalysis, protection or the like are located between the sensor layer and the fluid. The metal alloy of the sensor layer reacts with this hydrogen from the fluid, which diffuses through a protection layer, and builds a metal-alloy hydride system. The latter reaction is a reason for the change in dielectric properties/optical properties of the sensor layer when hydrogen is present, which is used for a hydrogen detection, or if desired the detection of other gases, in embodiments.

Next, some aspects relating to the optical sensing system for detecting hydrogen are described in more detail. The sensing system for detecting hydrogen comprises a light source. The optical sensing system has a sensor layer that changes its optical response, in particular its refractive index, depending on an amount of hydrogen present in the sensor layer. This means, a changing hydrogen partial pressure also leads to a change in the refractive index of the sensor layer.

As stated above, the amount of hydrogen can qualitatively be defined in terms of a partial pressure of hydrogen at the optical sensor, which is directly related to an amount of hydrogen (in ppm) dissolved in the insulation liquid. Thereby, the refraction index of the optical sensor expresses the amount of hydrogen, and can therefore be taken as a status indicator of the electrical equipment - as a growing hydrogen concentration is an early indication for potential fault of the electrical equipment, as described further above.

In aspects, an integral solution is provided to determine the hydrogen concentration dissolved in transformer oil, without having the need of an external reference measurement, such as for the intensity of the light source.

In aspects, an optical hydrogen sensor, and a respective hydrogen sensing system and method are provided. Basically, two different light intensities/light powers are taken as a measure for a hydrogen partial pressure at the sensor. The corresponding two light waves have, preferably, orthogonal linear polarization, but share the same optical source. They are sent along the same optical path to two respective detectors. Accordingly, the ratio of the two light powers is independent of variations of source light power and transmission changes of the common optical path. The two light waves are reflected, on their optical path, at an interface between a transparent substrate and a sensor layer under a non-normal angle of incidence, i.e., an angle of incidence larger than 0°. The reflection of both light waves at the interface is dependent on the dielectric properties of the sensor layer, which are in turn dependent - based on a hydrid-building process - on a hydrogen partial pressure at the sensor layer. The ratio between the reflected light intensities is thus indicative of the hydrogen partial pressure (or concentration) in the medium in contact with the sensor layer. A control unit calculates the hydrogen partial pressure in the medium based on stored values.

In embodiments, reflected light powers for typically both s- and p-polarized light vary with hydrogen uptake. The angle of incidence of the light on the sensor layer (with respect to the normal direction on the sensor layer) is chosen to be in the regime which encompasses a range from >0°, preferably around the Brewster angle, up towards - but not including, 90°, or in embodiments smaller than 90°, or - if defined - the critical angle of total reflection. Further details influencing the choice of the angle of incidence according to embodiments are provided further below.

According to an aspect, an optical gas sensing system, in particular the control unit thereof may further comprise a network interface for connecting the system to a data network, in particular a global data network. The data network may be a TCP/IP network such as the Internet. The system is operatively connected to the network interface for carrying out commands received from the data network. The commands may include a control command for controlling the system to carry out a task such as a measurement, a self-test, a calibration, or the like. In this case, the control unit is adapted for carrying out the task in response to the control command. The commands may include a status request. In this case, the control unit may be adapted for sending a status information to the network interface, and the network interface is adapted for sending the status information over the network in response to the status request. The commands may include an update command including update data. In this case, the control unit is adapted for initiating an update in response to the update command and using the update data.

According to further aspects, an electrical equipment with an insulation liquid is provided, wherein an optical sensor of the gas sensing system as described herein is immersed in the insulation liquid (i.e. partially immersed so that the optical sensor is at least in partial contact with the insulation liquid) or separated from the insulation liquid by a permeable membrane.

### Detailed Description of the Figures and Embodiments

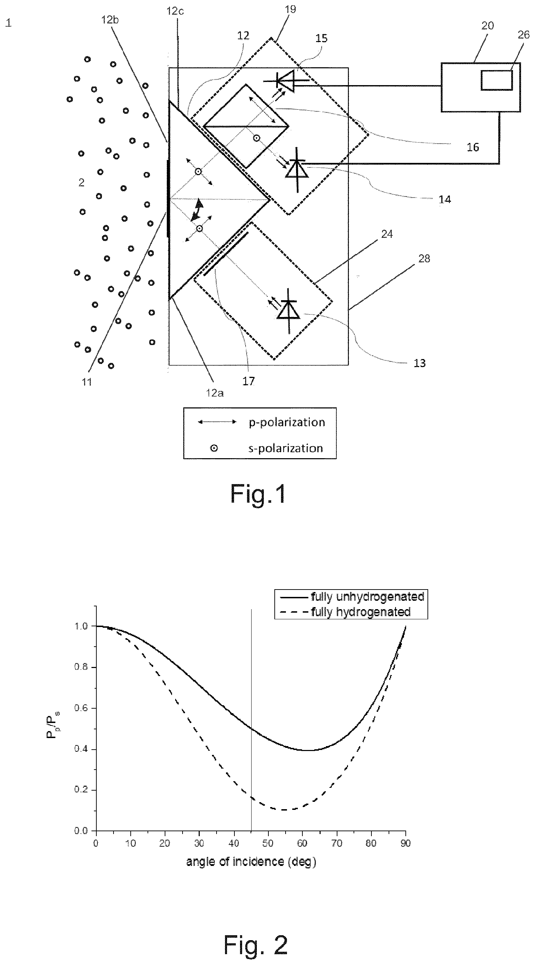

Fig. 1 shows an exemplary hydrogen sensing system 1 according to embodiments. It is suitable to detect or monitor hydrogen in a medium 2, such as a fluid, in particular in transformer oil used as an insulating medium in an electrical transformer.

The system has a light emitting unit 24 which emits polarized light. In the embodiments, the light emitting unit 24 includes a light source 13, which is exemplarily a LED in Fig. 1, and the non-polarized light from the LED is guided through a polarizing filter 17, preferably with principal axes aligned under 45° to the plane of incidence, which is exemplarily a thin-film polarizer. In Fig. 1, the polarized light is guided into an optical element 22 being exemplarily a triangle prism 12. In other embodiments, a differently shaped optical element 22 may be employed, which is laid out further below. The prism 12 has a first side face 12a, through which the polarized light is entering the prism 12. The prism has a sensing face 12b, which is configured to face the medium 2 to be monitored. A sensor layer 11 is attached to the outside of the sensing face 12b of the prism. As shown in Fig. 1, basically all elements of the sensing system 1 may be provided in a housing 28, typically except the control unit 20. Other light emitting units emitting light with two defined polarization components may be used in embodiments.

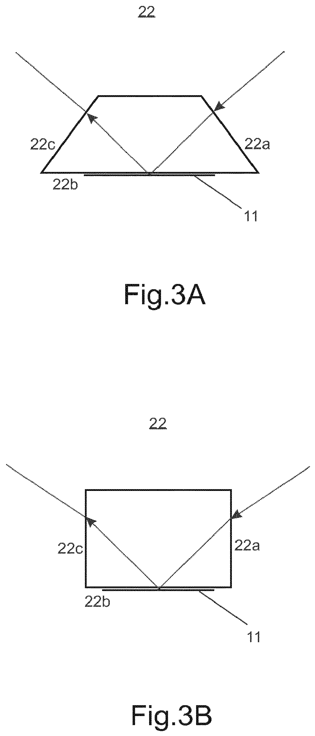

The sensor layer 11 is illuminated at its backside by the polarized light which has entered the prism 12 through the first side face 12a. The light is reflected at the interface between the sensor layer 11 and the sensing face 12b of the prism 12 and is thereby directed towards a second side face 12c of the prism. As is apparent from the exemplary embodiment shown in Fig. 1, the incoming light beam may have a normal direction with respect to the first side face 12a, and leaves the prism 12 also at a normal angle with respect to second side face 12c. In embodiments, the incidence angles at the side faces 12a, 12 c may deviate from 90°, which is laid out, e.g., with respect to Fig. 3. The light beam leaving the prism 12 through the second side face 12c is then split into two beams having orthogonal polarization with respect to each other. In the embodiment of Fig. 1, this is carried out by a polarizing beam splitter 16, which is exemplarily a polarizing beam splitter cube. Thus, the polarizing beam splitter 16 is arranged to receive light reflected by the sensor layer 11 through the second side face 12c of the prism 12. A first light detector 14 is arranged adjacent to a first output port of the polarizing beam splitter 16, and a second light detector 15 is arranged adjacent to a second output port of the polarizing beam splitter 16.

The signals from the first light detector 14 and the second light detector 15 thus are a measure for the intensities of the two light beams, having orthogonal polarization with respect to each other, which leave the polarizing beam splitter 16. The two signals are detected by a control unit 20, which is operatively connected to the first and second light detectors 14, 15.

In some embodiments, the polarizing beam splitter 16 and the two light detectors 14, 15 may be replaced by, for example, a single detector which is able to discriminate between different polarization types.

The control unit 20 is configured to determine a gas partial pressure in the medium 2 on the basis of the ratio of the signals from the first light detector 14 and the second light detector 15.

This is based on the following: The polarized light entering the prism 12 (or: the optical element 22) through the first side face 12a has, after having passed the polarizing filter 17, a defined ratio between the components of p-polarization and s-polarization. The ratio between the components of p-polarization and s-polarization is influenced during reflection of the light at the sensor layer 11 by the dielectric properties, in particular the refractive index, of the sensor layer 11. The dielectric properties of the sensor layer 11 are dependent on the hydrogen partial pressure in the medium 2 to be monitored.

According to the exemplary, non-limiting embodiment of Fig. 1, the sensing system 1 may comprise a sensor layer 11 formed as a sensor multilayer, which is deposited on the sensing face 12b being the hypotenuse of a prism 12 being an orthogonal triangle prism. The sensor layer 11 comprises a sensing layer of, e.g., 200 nm of Mg₅₂Ni_{20.5}Zr_{27.5} deposited on the prism 12, and a catalyst layer of, e.g., 50 nm of palladium deposited on the sensing layer. Molecular hydrogen is dissolved in the medium 2, which is exemplarily transformer oil, due to degeneration of the transformer oil. The molecular hydrogen is catalyzed into atomic hydrogen, so that the material of the sensing layer forms a hydride and thereby changes its dielectric properties, and thus its optical properties. In the following, it is assumed that the properties of the sensing layer defined by real (n) and imaginary part (k) of the refractive index change at 600nm from n = 1.33 and k = 2.09 in a fully un-hydrogenated state to n = 1.88 and k = 0.86 in a fully hydrogenated state.

The light source 13 is a light emitting diode (LED) with light emission around a wavelength of about 600 nm. The refractive index of the prism 12 amounts to n = 1.52, k = 0. A collimating lens (not shown in Figure 1) produces a collimated beam impinging on the first side face 12a, being the first cathetus of the prism 12, under normal incidence. A polarizing filter 17 being a thin film polarizer is arranged along the optical path between light source 13 and prism 12. The principle axis of the thin film polarizer is aligned under 45° to a plane of light incidence. Accordingly, the light beam before being reflected off the sensing face 12b of the prism 12, being the hypotenuse of the prism, is comprised to equal amounts of light polarized in the plane of incidence (p-polarization) and of light polarized perpendicular to the plane of incidence (s-polarization). Light of both former polarizations is reflected at the interface between the sensor layer 11 and the prism 12 with reflectivities corresponding to the optical parameters of the sensor layer 11 and the prism 12. A reflected light beam exits the prism 12 under normal incidence over the second side face 12c, being the second cathetus of the prism 12. In other embodiments, the light source 13 may comprise an SLED or a laser diode.

In embodiments, the real part of the refractive index of the prism 12 can be either smaller or larger than the real part of the refractive index of the sensor layer 11. The thin film polarizer 17 and polarizing beam splitter 16 can be directly attached to the prism 12, e.g, by an optical adhesive, or they can be mounted in spatial proximity to the prism 12. In the first case, they preferably have a refractive index close or equal to the refractive index of the prism 12.

The light beam is then split by the polarizing beam splitter 16 into s- and p-polarized light. The intensities (or powers) of the two light polarizations, Ps and Pp, are subsequently measured by the first light detector 14 and the second light detector 15, here formed as photodiodes. A ratio of Ps to Pp is calculated in the control unit 20, e.g. by employing a signal processor, and is used to determine the hydrogen partial pressure (concentration) in the medium 2.

It is notable that the ratio of Ps and Pp is independent of the power of the light source, and thus also the measurement precision is independent of short term or long term fluctuations of the light source. For illustration, the ratio of Ps to Pp is calculated from the Fresnel equations and the known optical constants as given above, which is shown in Fig. 2 as a function of the angle of incidence. In Fig. 1, the angle of incidence is shown as a double arrow between the normal direction on the sensing face 12b and the impinging light beam coming from the light source 24. A significant contrast between hydrogenated and un-hydrogenated state can be, as a non-limiting example, detected for an exemplary range for the angle of incidence from 35° to 65°. In Fig. 1, the angle is exemplarily about 45°, which is approximately the Brewster angle. In embodiments such as shown in Fig. 1, a choice of an orthogonal triangle prism as the optical element 12, 22 results in an angle of incidence of 45°. In the case of normal incidence of light entering and exiting the isosceles prism 12 as shown, the angle of incidence at the interface between sensor layer 11 and prism 12 is defined by the vertex angle of the prism. In embodiments, the optical element may have different shapes than shown in Fig. 1. Non-limiting examples include an isosceles triangle prism with a vertex angle, an isosceles triangle prism with a vertex angle between 30° and 60°, a right angle prism, an equilateral triangle prism, and a right isosceles triangle prism, a trapezoidal prism, a plane-parallel plate, or a cube-shaped optical element (see Fig. 3B). In principal, arbitrarily shaped transparent bodies may be used as the optical element, provided that light can enter them, be reflected at the boundary between a sensing face 12b, 22b and the sensor layer 11 at the chosen angle of incidence, and leave through a further side face 12c, 22c. Thereby, while not a necessity, it is preferred that the light can enter and exit the optical element with a normal angle towards the respective side faces of the optical element, in order not to interfere with the polarization properties of the light beam - while, if an oblique angle is chosen for at least one of the side faces 12a, 22a, 12c, 22c, the resulting influence on the polarization components of the light, which is well known to the skilled person, can be accounted for in the calculation process in the control unit 20.

Generally, according to embodiments, the reflected light powers for both s- and p-polarized light vary with hydrogen uptake. Preferred embodiments do not cover working at an angle of incidence on the sensor layer 11 which would result in a total reflection of the impinging light wave. This means also that typically, no surface plasmons are excited in the sensor layer 11. Instead, the angle of incidence may be chosen to be in the regime which encompasses a range from > 0° up towards - but not including - the critical angle of total reflection.

In some embodiments, part of, or all interfaces along the optical path between the first light detector 14, second light detector 15, prism 12, thin film polarizer 17, and polarizing beam splitter 16 are coated with anti-reflection coatings.

It goes without saying that other light polarizations, deviating from s- and p-polarization, may be employed as well. The calculation process has to be adapted in this case, which is a standard task for the skilled person. The same is valid for embodiments in which the optical element 22, and/or the arrangement of the different elements, is chosen so that the angle of incidence of the light on the first side face 12a, 22a, coming from the light source, is different from a normal direction, and/or the angle of incidence of the reflected beam out of the second side face 12c, 22c is different from a normal direction.

In Fig. 3A and 3B, two different shapes of optical elements 22 with first side faces 22a, sensing faces 22b with sensing layer 11, and second side face 22c are depicted (in the dimension perpendicular to the drawing plane, the typically uniform height of the optical element would be visible). It is understood that a variety of other shapes, having, e.g., also different number of side faces or even curves, may be employed, while the resulting system still falls under the scope of this disclosure.

According to embodiments, the optical element 22 (prism 12 in Fig. 1) typically comprises optical glass, preferably one of fused silica, quartz, BK-7, rutile, and GGG, or the like. The light emitting unit 24 comprises a polarizer 17, preferably a polarizing thin film arranged between the light source 13 and the optical element 22. The polarizing beam splitter 16 may typically comprise one of: a polarizing beam splitter cube (as exemplarily shown in Fig. 1), a Wollaston prism, a Glan-Thomson prism, or a Glan-Laser prism, or any combination of the former.

In embodiments, the sensor layer 11 may comprise palladium (Pd) or a palladium alloy, for example at least one of Pd-Au, Pd-Cu, Pd-Ag, wherein the sensor layer 11 may typically have a thickness from about 5 to 200 nm. Further, the sensor layer 11 may in embodiments also comprise a multilayer structure, comprising a sensing layer and a catalyst layer (not shown). The sensing layer may, e.g., comprise an alloy of Mg, Ni, and a metal M selected from the group including at least one of: zirconium, tantalum, and hafnium, while the ratio between Mg : Ni : M may be, for example: 0.4 - 0.6 : 0.1 - 0.4 : 0.1 - 0.40. The catalyst layer may comprise Pd or a Pd alloy. Further, in all embodiments, the sensor layer 11 may include a protective layer which shields the sensing layer, and which may comprise at least one of PTFE, PMMA, SiO2, aluminum oxide, or a multilayer combining two or more of the former. In the case of the sensor layer 11 being a multilayer, the sensing layer and the catalyst layer are arranged between the optical element (12, 22) and the protective layer.

According to embodiments, the above disclosed system may be employed in a method for monitoring a hydrogen concentration or hydrogen partial pressure in a medium 2. The method includes coupling light with a defined ratio between two polarization components into an optical element 22 through a first side face 22a of the optical element 22. The light is then reflected at an interface between a sensing face 22b of the optical element 22 and a sensor layer 11. The latter is in contact with a medium 2 to be monitored. The sensor layer 11 has a refractive index which is dependent on a hydrogen partial pressure in the medium 2.

The light reflected at the sensor layer 11 is then guided out of the optical element 22 through a second side face 22c. The reflected light is split into two partial beams with different polarization. The two partial beams are then guided onto a first light detector 14 and onto a second light detector 15, respectively. The two intensities are thus measured by reading out the first light detector 14 and the second light detector 15 with the control unit 20.

The control unit is typically configured to determine a (hydrogen) gas partial pressure in the medium 2 on the basis of the ratio of the signals from the first light detector 14 and the second light detector 15. This is carried out by taking into account that polarized light entering the optical element 22 through the first side face has a defined ratio between the components of p-polarization and s-polarization. The ratio between the components of p-polarization and s-polarization, which may, e.g., be expressed via the ratio between the intensities, or via the difference divided by the sum of both intensities, is subsequently influenced during reflection at the sensor layer 11 by the dielectric properties, in particular the refractive index, of the sensor layer 11. The dielectric properties are dependent on a hydrogen partial pressure in the medium 2 to be monitored, which is in physical contact with the sensor layer 11.

In embodiments, the control unit 20 typically comprises a database 26 or memory section comprising ratios of the components of p-polarization and s-polarization for a plurality of hydrogenation levels of the sensor layer 11, which are used to calculate the hydrogen partial pressure in the medium. The ratio may, e.g., be expressed by the ratio between the intensities, or the difference divided by the sum of both intensities.

It is understood that the described optical sensor system 1 has to be characterized prior to using it. In the characterization phase, data is obtained about the correlation between hydrogen partial pressure in the medium 2, temperature and the respective ratio between p-polarization and s-polarization. As such optical sensors can be reproduced with high precision, this might only be necessary for a prototype, and the obtained data may be reused in mass production. In embodiments, the medium 2 is a transformer oil being an insulation liquid in a power transformer or in an electrical installation or apparatus which comprises an insulation and/or cooling liquid.

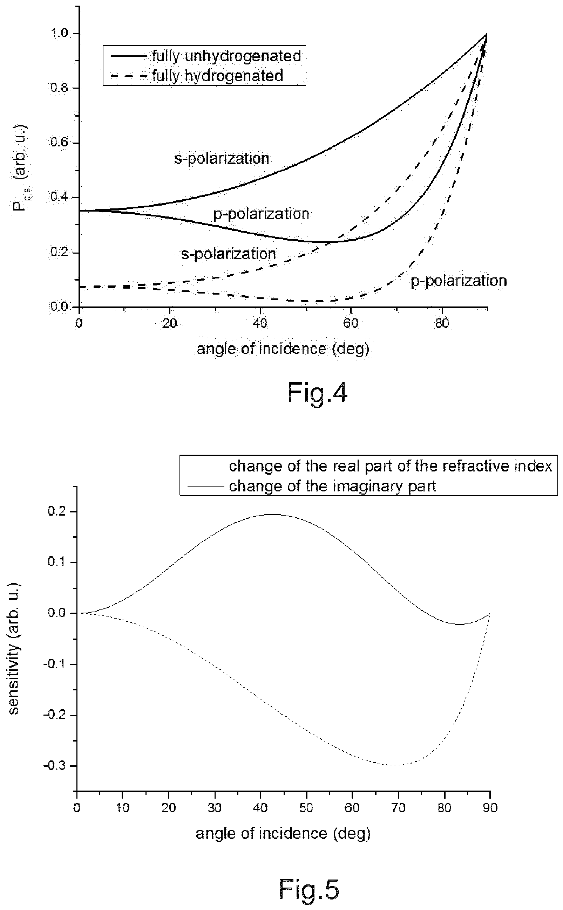

Fig. 4 depicts the change of the degree of reflection for both the p-polarization component and the s-polarization component of an exemplary combination of a prism 12 with a sensor layer 11 as shown in Fig. 1, at different angles of incidence. The solid line indicates reflection in a fully unhydrogenated state of the sensor layer, meaning in the absence of hydrogen in the surrounding of the sensor layer 11, respectively in the medium 2. The dashed line shows the reflection in a fully hydrogenated state, i.e., when the sensor layer is saturated with hydrogen. It is understood that the correlation between the actual hydrogen partial pressure in the medium 2 and the optical responses has to be initially characterized, the results of which may be stored as a look-up table in a memory of the control unit 20 for the ratio between p-polarization and s-polarization (or different polarization types, if chosen) at different hydrogen partial pressures.

Fig. 5 depicts the sensitivity of the sensor signal on relative change of the real part and the imaginary part of the refractive index of the sensor layer 11, or of the sublayer of the sensor layer being closest to the optical element 12, 22.

Although specific features of various embodiments of the invention may be shown in some drawings and not in others, this is for convenience only. In accordance with the principles of the invention, any feature of a drawing may be referenced and/or claimed in combination with any feature of any other drawing.

This written description uses examples to disclose the invention, including the best mode, and also to enable any person skilled in the art to practice the invention, including making and using any devices or systems and performing any incorporated methods. While various specific embodiments have been disclosed in the foregoing, those skilled in the art will recognize that the spirit and scope of the claims allows for equally effective modifications. Especially, mutually non-exclusive features of the embodiments described above may be combined with each other. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

## Claims

1. A hydrogen sensing system (1) for detecting hydrogen in a medium (2) to be monitored, the system comprising:
a light emitting unit (24) emitting light with two polarization components having a first intensity ratio between the polarization components,
an optical element (12, 22),
a sensor layer (11) which is attached, with a first face, to a sensing face (12b, 22b) of the optical element, wherein the sensor layer (11) is configured to be in contact, at a second face opposite to the optical element, with the medium (2), and to have a refractive index which is dependent on a hydrogen partial pressure in the medium at the sensor layer (11),
a light detection unit (19) configured to detect the intensity of each of the two polarization components of the detected light,
a control unit (20) operatively coupled to the light detection unit (19),
wherein light emitted by the light emitting unit (24) is guided into the optical element (12, 22) and is reflected at an angle of incidence larger than 0°at the boundary between the sensor layer (11) and the optical element (12, 22), and wherein the control unit (20) is configured to determine a gas partial pressure at the sensor layer (11) on the basis of a ratio of the intensities of the polarization components of the reflected light as detected by the light detection unit (19).

2. The system of claim 1, wherein the light emitting unit (23) comprises a polarizer (17), preferably a polarizing thin film arranged between a light source (13) and the optical element (12, 22).

3. The system of claim 1 or 2, wherein the polarizing beam splitter (16) comprises one of: a polarizing beam splitter cube, a Wollaston prism, a Glan-Thomson prism, and a Glan-Laser prism, a non-polarizing beam splitter with polarizers or thin film polarizeras attached to output ports.

4. The system of any preceding claim, with n1 being a real part of a refractive index of the optical element (12, 22) at the wavelength of operation, and n2 being a real part of the refractive index of either a single layer or of a sublayer of the sensor layer being closest to the optical element (12, 22) at the wavelength of operation,
wherein n1 is larger or smaller than the refractive index n2 of the sensor layer, and wherein the system is configured such that light impinges on the sensor layer, in the case of n2 < n1, under an angle of incidence smaller than a critical angle given by arcsin n2/n1.

5. The system of any preceding claim, wherein the reflection of the two polarization components is sensitive to changes of both the real part of the refractive index and the imaginary part of the refractive index of the sensor layer, and wherein preferably, the angle of incidence is configured to be in a range of +-10°, preferably +-5°, around the Brewster angle.

6. The system of any preceding claim, wherein the optical element (12, 22) further has a first side face (12a, 22a) through which polarized light is entering the optical element, and a second side face (12c, 22c) through which the reflected light leaves the optical element towards the light detection unit (19), and
- wherein the light detection unit comprises a polarizing beam splitter (16), being arranged to receive light reflected by the sensor layer (11) through the second side face of the optical element (12, 22), and a first light detector (14) arranged at a first output port of the polarizing beam splitter (16), and a second light detector (15) arranged at a second output port of the polarizing beam splitter (16),
- wherein the polarized light entering the optical element (12, 22) through the first side face has a defined ratio between two polarization components, which are preferably the components of p-polarization and s-polarization.

7. The system of any preceding claim, wherein the optical element (12, 22) comprises optical glass, preferably one of fused silica, quartz, BK-7, rutile, and GGG.

8. The system of any preceding claim, wherein the optical element (12, 22) is one of: a prism, an isosceles triangle prism, a prism, an isosceles triangle prism with a vertex angle between 30° and 60°, a right angle triangle prism, a right angle isosceles triangle prism, an equilateral triangle prism, a right isosceles triangle prism, a trapezoidal prism, and a plane-parallel plate. and wherein the light from the light emitting unit (24) preferably has a normal angle of incidence with respect to the first side face (12a, 22a) of the optical element (12, 22).

9. The system of any preceding claim, wherein the sensor layer (11) comprises at least one of:
a. a single layer comprising palladium or a palladium alloy, preferably at least one of Pd-Au, Pd-Cu, Pd-Ag, having a thickness from 5 to 200 nm; and
b. a multilayer structure, comprising sublayers being a sensing layer and a catalyst layer, wherein the sensing layer comprises preferably an alloy of Mg, Ni, and a metal M selected of the group of zirconium, tantalum, and hafnium, and wherein the catalyst layer preferably comprises Pd or a Pd alloy;
and wherein the sensor layer further comprises optionally a protective layer comprising at least one of PTFE, PMMA, SiO2, aluminum oxide, or a multilayer combining two or more of the former, wherein the sensor layer is arranged between the optical element (12, 22) and the protective layer.

10. The system of any preceding claim, wherein the light emitting unit (24) comprises a LED or a laser diode.

11. The system of any preceding claim, further comprising a network interface for connecting the system to a data network (72), wherein the control unit (70) is operatively connected to the network interface (71) for carrying out commands received from the data network, and/or to send measurement data to the data network (72).

12. A method of detecting hydrogen in a medium (2) to be monitored, comprising:
- providing a sensing system of any of claims 1 to 9;
- coupling light having two polarization components, having a first intensity ratio between the polarization components, into an optical element (12, 22);
- reflecting the light at a boundary between a sensing face (12b, 22b) of the optical element and a sensor layer (11) which is in contact with a medium (2) to be monitored and has a refractive index being dependent on a hydrogen partial pressure in the medium (2);
- guiding the reflected light out of the optical element (12, 22) and onto a light detection unit (19) configured to detect the intensity of each of the two polarization components of the detected light,
- determining, in a control unit (20), a gas partial pressure in the medium (2) on the basis of a ratio of the intensities of the two polarization components of the detected light.

13. The method of claim 10, wherein the control unit (20) comprises a database (26) comprising data on the ratio of the polarization components of, preferably, p-polarization and s-polarization, for a plurality of hydrogenation levels of the sensor layer (11), the ratio being employed to determine the hydrogen partial pressure in the medium (2).

14. The method of claim 10 or 11, wherein the monitored medium (2) is oil in an electrical installation.

15. The method of any of claims 10 to 12, wherein n1 is a real part of a refractive index of the optical element (12, 22) at the wavelength of operation, and n2 is a real part of the refractive index of either a single layer or of a sublayer of the sensor layer being closest to the optical element (12, 22) at the wavelength of operation,
wherein n1 is larger or smaller than the refractive index n2 of the sensor layer, and wherein the system is configured such that light impinges on the sensor layer, in the case of n2 < n1, under an angle of incidence smaller than a critical angle given by arcsin n2/n1.

16. The method of any of claims 10 to 13, wherein the reflection of the two polarization components is sensitive to changes of both the real part of the refractive index and the imaginary part of the refractive index of the sensor layer, and wherein preferably, the angle of incidence is configured to be in a range of +-10°, preferably +-5°, around the Brewster angle.

17. Use of a sensing system (1) comprising a light emitting unit (24), an optical element (12, 22) with a hydrogen-sensitive sensor layer (11) attached to a sensing face (12b, 22b) of the optical element, a light detection unit (19) and a control unit (20), to determine a hydrogen partial pressure in a medium (2) in contact with the sensor layer (11), by employing a measurement of the intensities of two polarization components of light reflected, at an angle of incidence larger than 0°, at a boundary between the optical element (12, 22) and the sensor layer (11), wherein a ratio of the intensities of the two polarization components of the reflected light is used to determine the hydrogen concentration in the medium (2).
